# EUROPÄISCHE PATENTSCHRIFT

(11) **EP 1 136 086 B1**
(45) Veröffentlichungstag und Bekanntmachung des Hinweises auf die Patenterteilung: **31.05.2006**
(21) Anmeldenummer: 01104970.7
(22) Anmeldetag: 01.03.2001
(51) Int. Cl.: A61L 29/12

(54) **PVC- und weichmacherfreie medizinische Arbeitsmittel**
PVC-and plasticiser-free medical articles
Articles médicaux exempts PVC ou plastifiants

(30) Priorität: 22.03.2000 DE 10014248
(43) Veröffentlichungstag der Anmeldung: 26.09.2001
(73) Patentinhaber: RAUMEDIC AG, 95233 Helmbrechts (DE)
(72) Erfinder: Kühlein, Georg, 95111 Rehau (DE); Ziembinski, Ralf, Dr., 95111 Rehau (DE)
(74) Vertreter: Rau, Albrecht

(56) Entgegenhaltungen:
- EP-A- 0 086 512
- US-A- 5 270 086
- US-A- 5 562 127

## Beschreibung

Die Erfindung betrifft medizinische Arbeitsmittel, welche im Extrusionsverfahren herstellbar sind, wie Schläuche, Rohre, Katheter und dergleichen, bestehend aus wenigstens einer Innenschicht und einer diese umgebende Außenschicht aus polymeren Werkstoffen.

Im bekannten Stand der Technik werden für derartige medizinische Arbeitsmittel hauptsächlich polymere Werkstoffe auf Basis von PVC verwendet, da dieser aufgrund der niedrigen Materialkosten und der auf Verkleben basierenden Konfektionstechnik für Anschlusselemente, wie z. B. Tropfkammem, Konnektoren, Unterdruckkissen u. a. m., ein gutes Preisleistungsverhältnis beinhalten.

Weitere Verwendung finden medizinische Arbeitsmittel, welche aus Polyurethan-Werkstoffen hergestellt sind. Mit diesen ist es zwar möglich, die bestehenden Konfektionstechniken zu verwenden, jedoch sind derartige medizinische Arbeitsmittel aufgrund des sehr hohen Materialpreises mit einem negativen Preisleistungsverhältnis behaftet, insbesondere bei Anwendungen im Dialyse- und Infusionsbereich. Eine weitere Möglichkeit aus dem bekannten Stand der Technik ist der Einsatz medizinischer Arbeitsmittel, welche aus Polyolefinen hergestellt sind. Diese haben zwar einen akzeptablen Materialpreis, lassen sich jedoch nicht mit der konventionellen Konfektionstechnik, wie beispielsweise das Verkleben zu handelsüblichen Dialysebestecken, für die Hämodialyse weiterverarbeiten. Die aus dem bekannten Stand der Technik resultierenden Materialien und Systeme weisen zum einen ein nachteiliges Preisleistungsverhältnis auf und sind zum anderen in der derzeitigen PVC-Diskussion mit den Schwerpunktthemen Weichmachermigration, Entsorgung wie z. B. die Salzsäurebildung bei der Verbrennung bzw. das Entstehen von Dioxinen und Furanen, negativ behaftet.

In der US 5,562,127 A sind Schläuche für den medizinischen Einsatz beschrieben. Diese bestehen aus einem Schichtverbund von polymeren Werkstoffen,
wobei zwei- und dreischichtige Schläuche dargestellt sind. Die Materialauswahl ist dabei so, dass der Einsatz von PVC vermieden wird. Bevorzugte Materialien zum Aufbau der medizinischen Schläuche der US 5,562,127 A sind weichmacherfrei. Für die Innenschicht wird z. B. Ethylen-Vinylacetat-Copolymer mit 28 Gew.-% Vinylacetatgehalt angegeben. Auch ein Polymetacrylat ist als mögliches Material für eine der Schichten angesprochen. Auch der Einsatz eines Copolymers mit einem Polyolefin ist erwähnt. Als Innenschicht-Materialien werden z. B. Polyethylen und Polypropylen genannt. Für die Außenschicht wird z. B. Polyurethan angegeben.

In der nachveröffentlichten WO 00/76 564 A1 ist ein medizinischer Mehrschicht-Schlauch beschrieben.

Die Erfindung hat es sich zur Aufgabe gestellt, die bekannten Nachteile des Standes der Technik zu beseitigen und medizinische Arbeitsmittel aufzuzeigen, welche wirtschaftlich herstellbar sind, welche die Patientengefährdung durch Weichmachermigration reduzieren und nach dem Gebrauch ohne Probleme entsorgt werden können.

Eine weitere Aufgabe wird daher gesehen, daß die aus dem Stand der Technik bekannten Konfektionstechniken weiter genutzt werden können.

Erfindungsgemäß wird dies durch die Kennzeichenmerkmale des Anspruches 1 gelöst. Weitere vorteilhafte Ausgestaltungen sind in den Unteransprüchen definiert.

Die erfindungsgemäßen medizinischen Arbeitsmittel sind dadurch gekennzeichnet, daß sie aus halogenfreien Werkstoffen hergestellt sind, daß die Außenschicht lösungsmittelverklebbar ist, daß die Innenschicht und die sie umgebende Außenschicht fest miteinander verbunden sind und das die Wandstärke der Außenschicht höchstens die Hälfte der Wandstärke der Innenschicht aufweist.

Durch die Verwendung von halogen- und weichmacherfreien Werkstoffen ist sichergestellt, daß ein während des Gebrauches der medizinischen Arbeitsmittel bezüglich Weichmachermigration keine Probleme auftreten. Damit ist vorteilhafterweise eine Patientensicherheit insbesondere bei leicht allergisch reagierenden Menschen gewährleistet. Weiterhin vorteilhaft wird gesehen, daß die erfindungsgemäß verwendete Außenschicht lösungsmittelverklebbar ist, so daß sich sämtliche auch aus der Verwendung von halogenhaltigen Werkstoffen bekannten Konfektionstechniken sowie die verwendeten Anschlußteile, Verbindungselemente etc. verwendet werden können.
Entsprechend den Anforderungen an die medizinischen Arbeitsmittel können die Wandstärken der Außenschicht als auch der Innenschicht so dimensioniert werden, daß zum einen eine feste Verbindung zwischen dem beispielsweise verwendeten Kathetern und den an ihnen befestigten Anschluß- bzw. Verbindungselementen gewährleistet ist, als auch die entsprechend geforderte mechanische Festigkeit und Knicksteifigkeit bei der Verwendung im sensiblen Bereichen.

Bei den erfindungsgemäßen medizinischen Arbeitsmitteln wird weiterhin vorteilhaft gesehen, daß die Innenschicht gleichzeitig die haftvermittelnde Schicht ist, so daß die feste Verbindung zwischen der Innen- und den entsprechenden Außenschichten jederzeit gewährleistet ist.

Für die Innenschicht der erfindungsgemäßen medizinischen Arbeitsmittel kann ein Ethylenvinylacetatcopolymer mit wenigstens 10 % Vinylacetat eingesetzt werden. Weiterhin vorteilhaft ist die Verwendung eines Ethylenmethylacrylates mit wenigstens 10 % Methylacrylat. Durch die Verwendung derartiger polymerer Werkstoffe kann vorteilhafterweise erreicht werden, daß die durch das Lumen der medizinischen Arbeitsmittel zu transportierenden Flüssigkeiten nicht beeinträchtigt werden und Wechselwirkungen wie beispielsweise Diffusion in beide Richtungen verhindert wird.

In einer weiteren vorteilhaften Ausgestaltung der erfindungsgemäßen hauptmedizinischen Arbeitsmittel wird für die Innenschicht ein Polyolefin verwendet. Dies hat den Vorteil, daß aufgrund der Materialpreise sich ein positives Preisleistungsverhältnis für die herzustellenden medizinischen Arbeitsmittel ergibt.

In einer weiteren vorteilhaften Ausführungsform kann für die medizinischen Arbeitsmittel ein thermoplastisches Elastomer verwendet werden, welches beispielsweise aus der Gruppe der elastomermodifizierten Polypropylene stammt.
Dieses gewährleistet wiederum, ebenso wie ein thermoplastisches Elastomer aus der Gruppe der elastomermodifizierten Polyethylene, daß die medizinischen Arbeitsmittel eine hohe Flexibilität bei ausreichender Biegesteifigkeit und erforderlicher Knicksteifigkeit besitzen. Durch die unterschiedliche Modifizierung der thermoplastischen Elastomere können die medizinischen Arbeitsmittel an den jeweiligen Anwendungsfall optimal angepaßt werden, so daß beispielsweise das Lumen von Schläuchen, Kathetern und dergleichen im Anwendungsfall durch ein Abknicken nicht verschlossen wird.

Aufgrund der Kombinationsmöglichkeiten der Innen- und Außenschichten ist es hier wiederum möglich, mit den erfindungsgemäßen medizinischen Arbeitsmitteln eine den speziellen Anforderungen gemäße Einstellung der Eigenschaften zu erreichen.

Aufgrund der Verwendung von Copolymeren aus Styrol und Butadien mit wenigstens 10 % Styrol für die Außenschicht der erfindungsgemäßen medizinischen Arbeitsmittel können die aus dem Stand der Technik bekannten Konfektionstechniken und Werkzeuge weiterverwendet werden. Das Preisleistungsverhältnis derart hergestellter medizinischer Arbeitsmittel hebt sich positiv mit denen aus dem bekannten Stand der Technik hergestellten ab.

Durch den Verzicht auf halogenhaltige Werkstoffe bei der Herstellung der erfindungsgemäßen medizinischen Arbeitsmittel können zum einen die bekannten Probleme der Entsorgung derartiger medizinischer Arbeitsmittel, die nach dem Erstgebrauch zur Diskussion steht, gelöst werden, während weiterhin eine mögliche Weichmachermigration und damit verbundene Patientengefährdung absolut ausgeschlossen ist.

Im Rahmen der erfindungsgemäßen medizinischen Arbeitsmittel liegt es außerdem, dass die Innenschicht und die Außenschicht durch das Verfahren der Coextrusion herstellbar sind, wobei auch Verfahren wie Lackieren, Gießen oder Tauchen zur Herstellung derartiger medizinischer Arbeitsmittel realisierbar sind.

Im Folgenden sollte an Ausführungsbeispielen, welche die Erfindung nicht einschränken, diese näher beschrieben werden.

Es zeigen:
- Figur 1:: Schnittdarstellung durch einen 2-schichtigen Schlauch.
- Figur 2:: Schnittdarstellung durch ein 3-schichtiges Rohr.

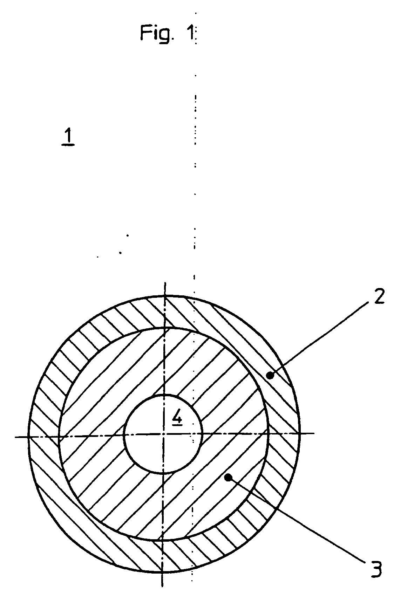

In Figur 1 ist ein Schnitt durch einen Schlauch 2 dargestellt.

Dieser für Katheter, Dialysebestecke, Drainagen, Infusionsbestecke u. a. m. verwendbare Schlauch ist derart aufgebaut, dass das Lumen 4 des Schlauches 1 durch eine Innenschicht 3 aus einem Ethylenvinylacetatcopolymer umgeben ist.

Die Innenschicht 3 wird vollumfänglich von einer Außenschicht 2 umgeben,
wobei die Innenschicht 3 und die Außenschicht 2 einstückig miteinander verbunden sind. In diesem Ausführungsbeispiel weist die Wandstärke der Außenschicht 2 etwa 1/3 der Wandstärke der Innenschicht 3 auf.

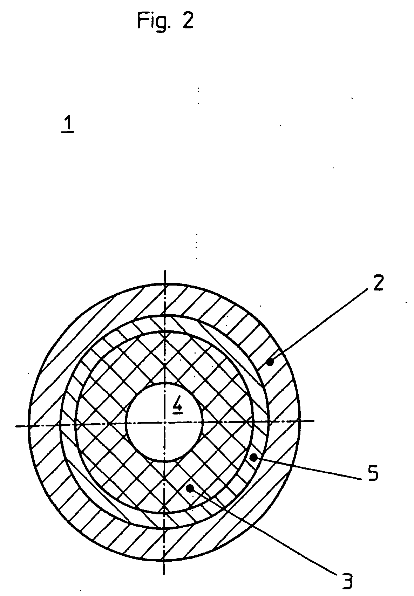

In Figur 2 ist die Schnittdarstellung durch ein Rohr 1 der erfindungsgemäßen medizinischen Arbeitsmittel dargestellt. Das Lumen 4 wird von einer Innenschicht 3 aus einem Polyethylen umgeben, wobei auf der Innenschicht 3 vollumfänglich eine Zwischensicht 5 aus einem Ethylenvinylacetatcopolymer aufgebracht ist. Diese Zwischenschicht 5 weist in etwa eine Wandstärke von ¼ der Innenschicht 3 auf und dient als zusätzliche haftvermittelnde Schicht zur Außenschicht 2. Die Außenschicht 2 ist ebenso vollumfänglich auf die Zwischenschicht 5 aufgebracht, wobei die Innenschicht 3, die Zwischenschicht 5 und die Außenschicht 2 fest und unlösbar miteinander verbunden sind.

## Patentansprüche

1. Katheter, Schläuche oder Rohre für den Einsatz in der Medizin, bestehend aus einem Schichtverbund von halogenfreien, weichmacherfreien polymeren Werkstoffen, wobei die Innenschicht gleichzeitig als haftvermittelnde Schicht dient, wobei die Wandstärke der Außenschicht höchstens die Hälfte der Wandstärke der Innenschicht aufweist und wobei die Innenschicht ausgewählt ist aus der Gruppe:
- Copolymeres aus Ethylen und Vinylacetat mit mindestens 10 % Vinylacetatgehalt,
- Copolymeres aus Ethylen mit Methylacrylat mit mindestens 10 % Methylacrylatgehalt,
- Elastomer-modifiziertes Polypropylen,
- Elastomer-modifiziertes Polyethylen,
**dadurch gekennzeichnet, dass** die Außenschicht aus Styrol-Butadien-Copolymeren mit wenigstens 10 % Styrol ist.

## Claims

1. Catheters, hoses or tubes for use in medicine, comprising a composite of layers of polymeric materials free from halogen and free from plasticizers, the interior layer simultaneously serving as an adhesive layer, with the wall thickness of the exterior layer maximally amounting to half the wall thickn ess of the interior layer and with the interior layer being selected from the group:
- copolymers of ethylene and vinyl acetate with a content of at least 10% of vinyl acetate,
- copolymers of ethylene with methyl acrylate with a content of at least 10% methyl acrylate,
- elastomer-modified polypropylene,
- elastomer-modified polyethylene,
**characterized in that** the exterior layer consists of styrene butadiene copolymers with at least 10% styrene.

## Revendications

1. Cathéters, tuyaux ou tubes destinés à être utilisés dans la médecine, constitués d'un composite de couches en matériaux polymères exempts d'halogènes et exempts de plastifiants, la couche intérieure servant simultanément de couche d'adhérence, l'épaisseur de la paroi de la couche extérieure étant au maximum la moitié de l'épaisseur de la paroi de la couche intérieure, et la couche intérieure étant choisie parmi le groupe
- des copolymères d'éthylène et d'acétate de vinyle avec une teneur en acétate de vinyle d'au moins 10%,
- des copolymères d'éthylène et de méthylacrylate avec une teneur en méthylacrylate d'au moins 10%,
- des polypropylènes à élastomères modifiés,
- des polyéthylènes à élastomères modifiés,
charactérisés en ce que la couche extérieure est en copolymère styrène butadiène contenant au moins 10% de styrène.
